# EUROPEAN PATENT APPLICATION

(11) **EP 1 050 280 A1**
(43) Date of publication of application: **08.11.2000**
(21) Application number: 99201412.6
(22) Date of filing: 05.05.1999
(51) Int. Cl.: A61B 19/04

(54) **Modified rubber latex with a reduced allergen activity and method for preparing said latex**

(71) Applicant: Budev Medical B.V., 5271 AT St. Michielsgestel (NL)
(72) Inventor: Jeekel, Johannes, NL-3062 HM Rotterdam (NL); Paping, Max Gregor, NL-5271 AT St.Michielsgestel (NL)
(74) Representative: Van Someren, Petronella F. H. M.

(57) **Abstract**

The present invention relates to rubber latex comprising an amount of starch, which rubber latex has a reduced allergen activity as compared to the same rubber latex without starch. In addition, the invention relates to a rubber latex article comprising said rubber latex, wherein at least the surface contacting the skin of the user is fabricated from said rubber latex. The invention futher relates to a method for reducing the allergen activity of rubber latex comprising incorporating an amount of starch in the rubber latex.

## Description

The present invention relates to rubber latex with a reduced allergen activity. The invention further relates to a method for preparing said rubber latex, and to medical and non-medical articles comprising said rubber latex.

Natural rubber latex is processed almost exclusively from the sap of the Hevea Brasilliensis tree (>99%), which is commonly found in Africa and Southeast Asia. Rubber workers collect the sap, a milky white dispersion known as liquid latex, by cutting deep strips into the bark of the tree. The liquid latex is an emulsion of rubber particles (cis-1,4,-polyisoprene) with diameters ranging from 5 nm to 3 µm (<d> = 0.25-0.8 µm) in an aqueous serum. The rubber particles are coated with a negatively charged layer of proteins, lipids and phospholipids that provide the structural integrity and stability of the dispersion.

For the manufacture of natural rubber products, such as latex rubber gloves, the starting material is the concentrated latex. The gloves are manufactured by dipping porcelain or glass moulds into the liquid latex. This can be achieved by dipping the moulds in a coagulating salt (calcium alginate) and then dipping them into a prevulcanized latex concentrate, yielding film thicknesses between 0.2 and 0.8 mm, or by dipping the moulds several times in the latex, and crosslinking the gloves afterwards. In the second method the films are not allowed to dry completely between dips in order to ensure homogeneous film formation. One dip accounts for approximately 0.05 mm. The final rubber product contains 93-96% polyisoprene and up to 3% protein by weight.

As a consequence of the increasing use of natural rubber articles, such as for example surgical gloves, the occurrence of latex allergy in hospital personnel and patients has become a major problem. Thus, more and more people are using surgical or examination gloves made from natural rubber latex containing a high level of proteins, which are the cause of the latex allergies. In particular, health care personnel and patients have shown a growing sensitivity to natural rubber products. The current estimate of healthcare workers being allergic to natural rubber gloves ranges between 10 and 20%. This phenomenon has been attributed to the recent dramatic rise in the use of latex gloves by medical, dental and auxiliary personnel for the protection against AIDS and hepatitis viruses. Although the allergic reactions are most obvious with respect to natural rubber gloves, a large number of other natural rubber articles are on the market, like balloons, condoms, footwear, clothing, adhesives, carpet backing etc. resulting in latex allergies as well. The problem of sensitivity to latex is therefore not restricted to (surgical) gloves.

The clinical manifestations of immediate hypersensitivity to latex usually arise from direct contact with natural rubber, but may also result from inhalation of airborne latex allergens. The symptoms and signs may be localized or generalized urticaria (development of wheals, flares and hives), angioedema, rhinitis, conjunctivitis, asthma, tachycardia and/or anaphylactic shock (increased heart beat rate, lowered blood pressure and possible loss of consciousness).

Allergy to latex is a typical example of an immunologically-mediated immediate hypersensitivity reaction, which is induced by allergenic proteins in the latex and is mediated by IgE antibodies. This reaction is known as a Type I allergy.

There are over 240 polypeptides in natural rubber latex, as detected by two dimensional electrophoresis. The protein concentration of a native latex sap was reported to be 16.53 mg/ml. A quarter of these proteins is associated with the rubber particles, while the rest is present in the non-rubber fractions. The number of allergenic polypeptides/proteins identified as allergens (in humans) ranges from 11 to 57.

A number of allergenic proteins have recently been detected in latex sap, ammoniated latex and extracts of rubber gloves. Thus, a trypsin-sensitive allergen was demonstrated with a molecular weight around 30 kDa. In addition, it has been found that the Rubber Elongation Factor (REF = 58 kDa), which plays an important role in the polymerization of the polyisoprene chains, is a major allergen in latex. Of the major allergen prohevein (20 kDa) and the prohevein C-domain (14 kDa) it was found that its N-terminal 43-amino acid fragment hevein carries the main IgE-binding epitope. Hevein is the most predominant protein in natural rubber latex and has chitin binding properties. A 23 kDa polypeptide, which shows some amino acid sequences similar to the REF also shows allergen activity. Furthermore, lysozyme (27 kDa), which is related to the defense-related proteins in rubber latex, a 46 kDa and a 36 kDa protein are found to be allergens.

The latex proteins are believed to dissolve in the body sweat inside the gloves and are then absorbed through the skin. The onset of latex sensitization is insidious in nature and is progressive. It occurs slowly, sometimes over a period of many years, as the body is repeatedly exposed to latex and develops an immunologic memory to the proteins. The presence of latex specific IgE antibodies in the bloodstream precedes the development of clinical symptoms by months or years. It is not known what level of protein is required to actually sensitize an individual. Because of this no regulation exists for limiting the amount of allergenic protein that a product may contain.

In addition, most latex gloves are manufactured with a corn starch powder to facilitate donning. The allergenic proteins adhere to the donning powder, which may become airborne when the gloves are snapped on and off. As a result many healthcare workers inhale the protein-laden powder over a period of several years and thus may develop latex sensitivity.

Chemicals which are added to the latex prior to processing may also cause a severe rash and irritation. However, reaction to these chemicals is most commonly a Type IV allergy. Symptoms for Type IV allergy develop within 24 to 72 hours of exposure.

In order to measure the sensitivity to latex a number of allergy tests are available. The most reliable test is the skin prick test, in which a person is exposed to latex or latex extract via contact with the skin. Afterwards the reaction of the exposed skin is monitored. The latex RadioAllergoSorbent Test (RAST) is available for the in vitro detection of latex IgE antibodies (Latex, k82, Pharmacia Diagnostics), but is less sensitive than skin prick tests. In addition, a new latex-specific fluorescent enzyme immunoassay for the detection of latex specific IgE antibodies has been brought on the market (Pharmacia CAP System, PCS).

The in vitro assays show considerable variation in the total protein and allergen content of different glove brands. Furthermore, the amount of protein eluting from a glove depends on the method used and does not always correlate with the allergenicity in skin prick tests, indicating that the total protein measurement is not a sufficient method to monitor the allergenic properties of latex gloves.

In an attempt to reduce the allergenic effect of the allergens in gloves, the gloves are run through a chlorine wash process, known as leaching, after they are dipped and dried, to remove the proteins which are responsible for the allergic reactions. However, in efforts to speed up production and meet increasing demands, glove manufacturers may fail to adequately wash the gloves. Steam sterilization of the gloves further decreases the protein level.

The activity of allergens in latex can also be reduced by treatment with an alkaline potassium hydroxide solution. However, to reduce the allergenic effect of the latex an extra step in the production process is needed. In addition, the gloves will be more costly.

Another option is the use of latex-free gloves. These gloves can be made of neoprene, styrene butadiene block copolymer or styrene ethylene butadiene styrene block copolymer. However, these non-latex gloves often have inferior barrier properties and often are found to lack the comfort and fit of natural rubber latex gloves. Furthermore, they are less environmental-friendly as the energy required to produce them is 7-11 times more than is the case of natural rubber and they are generally not biodegradable. In addition, except for vinyl, the synthetic gloves are more costly.

Alternative methods to remove or inactivate the allergens in the latex are described in US 5,563,241 in which the rubber latex is contacted with an anion exchange resin. Subsequently, the protein-resin complex is removed from the latex. US 5,691,446 relates to a method of dipping the dried rubber product in a chemical substance that inactivates the allergens on the surface. Again, extra steps are needed for the manufacturing of latex articles. In US 5,777,004 proteases are added to the liquid latex for denaturation of the allergenic proteins. These proteases, however, may be the cause of allergic reactions themselves.

As a result of the high incidence of latex allergies the use of latex articles, such as surgical gloves, has been restricted or even banned from hospital environments, indicating the significance and impact of the problem of latex allergies.

The object of the present invention is therefore to reduce the allergen activity of natural rubber latex in order to reduce the incidence of latex allergies.

This is achieved by the present invention by providing a rubber latex comprising an amount of starch, such that the allergen activity of said rubber latex is reduced as compared to the same rubber latex without starch.

In the research that led to the present invention the effect of incorporating starch in rubber latex was investigated. It has thus been shown that by incorporating a small amount of starch in the rubber latex the allergen activity of said rubber latex can be reduced. The starch can form both physical and chemical bonds with the amino and acid groups of the proteins, thus binding potentially allergenic proteins.

Sources for the starch as used in the invention are starch preparations, which generally comprise starch and a small amount of other constituents, such as proteins. According to the present invention, preferably low-protein, colloidal starches are used.

According to the invention, the "allergen activity" of rubber latex refers to the amount of water-soluble allergens in extracts made from said rubber latex. Thus, in order to measure the allergen activity of rubber latex, extracts are made from rubber latex samples (as described in Example 1) and the amount of water soluble-allergens in these extracts is determined using a Latex Elisa for Antigenic Proteins (LEAP) test (Beezhold, The Guthrie Journal 61, 77-81, 1992). It has been shown that by adding small amounts of starch the allergen activity of the latex rubber samples (i.e. the amount of water-soluble allergens in a rubber latex extract) is significantly decreased as compared to the same rubber latex without starch, thus resulting in a reduced incidence of allergic reactions in persons contacting said rubber latex.

Comfort tests have shown that the use of starch concentrations of less than 10 w% do not have a negative effect on the mechanical properties of the samples. When more starch is added, the rubber samples are too stiff in order to be used in rubber articles, such as gloves.

According to a preferred embodiment of the present invention, the rubber latex comprises an amount of starch for reducing the allergen activity of rubber latex such that the allergen activity of said rubber latex is maximally 50%, preferably maximally 40%, more preferably maximally 30%, most preferably maximally 25% of the allergen activity of rubber latex without starch, as measured by a latex ELISA for antigenic proteins.

In particular, according to the present invention the rubber latex preferably comprises an amount of starch for reducing the allergen activity of rubber latex such that the allergen activity of said rubber latex is maximally 20%, preferably maximally 15%, more preferably maximally 10%, most preferably maximally 5% of the allergen activity of rubber latex without starch, as measured by a latex ELISA for antigenic proteins. The allergen activity of the rubber latex according to the invention thus is significantly reduced as compared to the currently used rubber latex without starch.

Preferably, the used starch is a modified starch. Methods for obtaining modified starch are for example described by Wurzburg (in: Modified starches: Properties and Uses, 1986; CRC Press Inc, Eds, Bocaraton, Florida, USA). However, according to the invention modified starch is preferably obtained by gelatinizing the starch in an extruder, and crosslinking the starch with glyoxal as described in the co-pending European patent application No. 99200203.0 and Example 1 of the present application. Particles of the modified starch (100-200 nm) are dispersed in water to obtain a 10 w% dispersion, which is then mixed with liquid rubber latex.

According to the present invention various starches can be used, such as for example potato starch, Tapioca, waxy corn starch and waxy rice starch.

The invention further relates to a method for reducing the allergen activity of rubber latex comprising incorporating an amount of starch in the rubber latex. In particular, the invention relates to a method for reducing the allergen activity comprising incorporating an amount of starch in the rubber latex such that the allergen activity of said rubber latex is maximally 50%, preferably maximally 40%, more preferably maximally 30%, most preferably maximally 25% of the allergen activity of rubber latex without starch, as measured by a latex ELISA for antigenic proteins.

According to a particularly preferred embodiment of the invention the method for reducing the allergen activity of rubber latex comprises incorporating an amount of starch in the rubber latex such that the allergen activity of said rubber latex is maximally 20%, preferably maximally 15%, more preferably maximally 10%, most preferably maximally 5% of the allergen activity of rubber latex without starch, as measured by a latex ELISA for antigenic proteins.

The fact that the method according to the invention involves low material costs and can be easily implemented in the existing glove manufacturing processes, without significant investments, is an important advantage of the present invention.

Furthermore, the invention relates to rubber latex articles, such as surgical gloves, condoms, inflatable balloons etc., comprising the rubber latex of the invention, wherein at least the surface contacting the skin of the user is fabricated from the modified rubber latex.

The invention further relates to the use of starch for reducing the allergen activity of rubber latex, and to the use of the rubber latex according to the invention for the manufacture of rubber latex articles.

By using the rubber latex of the present invention for the manufacture of rubber latex articles the incidence of allergic reactions to latex can be significantly reduced. This is particularly important for health care personnel, such as dental, medical and auxiliary personnel, as they are at the highest risk for developing severe latex allergies.

The present invention is further illustrated by the following example.

### EXAMPLE

### EXAMPLE 1

### Preparation of the rubber latex of the invention

A concentrated natural rubber latex was delivered in a drum with a total solid content of approximately 62%, which was modified by the incorporation of a modified strach as allergen-reducing compound.

The starch was a modified native potato starch. An extruder was used to gelatinise and crosslink the starch with glyoxal. A mixture of potato starch and glycerol (87:13) was fed into a twin screw extruder. After gelatinisation, a crosslinker (1-4 w% glyoxal) was injected and the starch was crosslinked. The extrudate thus obtained was dried, ground and dispersed in water, resulting in a 10% dispersion of starch particles (100-200 nm). The liquid latex was mixed with the starch dispersion. The weight fraction of the starch in the dried sample ranged from 0-30%. Fractions of 1-2% gave however the best results.

After the compounds were mixed, test tubes were dipped in the latex for the production of latex specimens. The number of dips ranged between one and four, and the drying temperature was 50°C. After preparation, the dried rubber samples were powdered with native cornstarch in order to reduce the stickiness of the rubber.

### Sample characterization

As described earlier, natural rubber latex specimens were made by dipping test tubes in the liquid rubber latex of the invention. This resulted in condom shaped rubber samples (samples: NR00S, NR01S, NR02S).

Since the addition of starch may modify the mechanical properties of the rubber, it was investigated whether the elasticity and strength of the modified rubber was changed after the incorporation of the starch. In addition, both total protein content and allergen content of the samples were determined.

The total amount of soluble proteins was measured using turbidity measurements. A 10% (w/v) extract was made from small pieces cut from the rubber samples in a phosphate buffer with 0.03% HSA and 0.5% phenol. After one hour of shaking, the extracts were centrifuged for 10 min. at 2000g. The supernatant was filtered over a Millipore 0.22 µm filter. The extracts were stored at -22°C. A small amount of the extract was preincubated in an alkaline solution containing EDTA. Benzethonium chloride (Boehringer Mannheim U/CSF) was then added, producing a turbidity which was read at 505 nm.

The amount of water-soluble allergenic proteins in the rubber latex extracts was determined using the Latex ELISA for Antigenic Proteins (LEAP) as used in the Allergology department of the Academic Hospital of Rotterdam (Beezhold, The Guthrie Journal 61, 77-81, 1992).

### Results

### Wearing comfort:

The comfort tests showed that after addition of starch to the rubber latex, the elasticity of the dipped samples was reduced. This increment of the stiffness was most notable for samples having a starch content higher than 10%. The elasticity modulus of the 10% starch-latex samples was three times higher than that of the non-modified ones. Furthermore, the surface of the samples became less smooth with increasing starch load. From this it was concluded that the mechanical properties of the samples having a starch load up to 10% were comparable to the non-modified samples.

### Protein content:

In table 1 the results of the addition of 1 and 2 % of modified potato starch are shown. From this table it can be concluded that the total amount of soluble proteins did not depend on the amount of starch added. This seems strange, since a dilution effect should be expected. However, the majority of measured proteins originate from the 0.03% HSA in the phosphate buffer. Furthermore, it is known that the starch preparation which is used itself also contains a small amount of proteins. In addition, it is also not inconceivable that the starch absorbs proteins in the liquid latex and thus induces an enhanced protein content in the final samples.

**Table 1:**

| Results of the comfort, protein and allergen tests on the modified natural rubber samples | | | | | | |
|---|---|---|---|---|---|---|
| sample | starch w% | dips | weight (g) | comfort | protein (g/l) | allergen (µg/ml) |
| NR00S1 | 0 | 1 | 0.54 | + | 0.31 | 1.77 |
| NR00S_2 | 0 | 2 | 0.74 | + | 0.32 | 2.15 |
| NR00S_3 | 0 | 3 | 1.02 | + | 0.33 | 1.42 |
| NR01S_1 | 1 | 1 | 0.26 | + | 0.32 | 0.66 |
| NR01S_2 | 1 | 2 | 0.69 | + | 0.34 | 0.77 |
| NR01S_3 | 1 | 3 | 0.90 | + | 0.33 | 0.44 |
| NR02_1 | 2 | 1 | 0.32 | + | 0.32 | 0.38 |
| NR02_2 | 2 | 2 | 0.60 | + | 0.33 | 0.74 |
| NR02_3 | 2 | 3 | 0.95 | + | 0.32 | 4.31 |
| Romed Baxter Nu Tex Biogel Comform | | | | | | > 5.4 |

### Allergen activity:

The most significant results of the sample characterisation are listed in the last column of table 1. In this column the allergen concentrations in µg per ml extract are given. The numbers >5.4 indicate that the allergen content is too high to be measured accurately using the method described earlier.

When the 1 % and 2% starch samples were compared to the 0% sample, a decrease in the amount of water-soluble allergens of 60-75% was observed. This indicates that the addition of small amounts of starch to the liquid rubber latex before processing reduced the allergen activity of the rubber latex of the invention to maximally 25% to 40% of the allergen activity of rubber latex without starch. The allergens are absorbed at the surface of the starch particles which are subsequently fixed in the rubber matrix, resulting in a decrease of the allergen activity of natural rubber latex.

In the last row of table 1, the results of five different brands of glove are listed. The allergen content of all five brands exceeds 5.4 µg/ml extract. This means that even the 0% starch sample gave better results than the commercial brands. This may be due to the industrial processing of the gloves. The samples as described in this example were dried at 50°C. It is possible that this drying step already partly denaturises the allergenic proteins.

## Claims

1. Rubber latex comprising an amount of starch, which rubber latex has a reduced allergen activity as compared to the same rubber latex without starch.

2. Rubber latex according to claim 1 **characterized in that** the rubber latex comprises an amount of starch for reducing the allergen activity of latex such that the allergen activity of said rubber latex is maximally 50%, preferably maximally 40%, more preferably maximally 30%, most preferably maximally 25% of the allergen activity of rubber latex without starch, as measured by a latex ELISA for antigenic proteins.

3. Rubber latex according to claim 1 or 2 **characterized in that** the rubber latex comprises an amount of starch for reducing the allergen activity of latex such that the allergen activity of said rubber latex is maximally 20%, preferably maximally 15%, more preferably maximally 10%, most preferably maximally 5% of the allergen activity of rubber latex without starch, as measured by a latex ELISA for antigenic proteins.

4. Rubber latex according to claim 1, 2 or 3 **characterized in that** the starch is a modified starch.

5. Rubber latex according to claim 4 **characterized in that** the modified starch is obtainable by gelatinising the starch in an extruder and subsequently crosslinking the starch with glyoxal.

6. Rubber latex according to any of the claims 1-5 **characterized in that** the starch is potato starch, Tapioca, waxy corn starch or waxy rice starch.

7. Method for reducing the allergen activity of rubber latex comprising incorporating an amount of starch in the rubber latex.

8. Method according to claim 7 **characterized in that** the amount of starch that is incorporated in the rubber latex is such that the allergen activity of said rubber latex is maximally 50%, preferably maximally 40%, more preferably maximally 30%, most preferably maximally 25% of the allergen activity of rubber latex without starch, as measured by a latex ELISA for antigenic proteins.

9. Method according to claim 7 or 8 **characterized in that** the amount of starch that is incorporated in the rubber latex is such that the allergen activity of said rubber latex is maximally 20%, preferably maximally 15%, more preferably maximally 10%, most preferably maximally 5% of the allergen activity of rubber latex without starch, as measured by a latex ELISA for antigenic proteins.

10. Method according to claim 7, 8 or 9 **characterized in that** the starch is a modified starch.

11. Method according to claim 10 **characterized in that** the modified starch is obtainable by gelatinising the starch in an extruder and subsequently crosslinking the starch with glyoxal.

12. Method according to any of the claim 7-11 **characterized in that** the starch is potato starch, Tapioca, waxy corn starch or waxy rice starch.

13. Rubber latex article comprising rubber latex according to claims 1-6, wherein at least the surface contacting the skin of the user is fabricated from the said rubber latex.

14. Rubber latex article according to claim 13 **characterized in that** the article is a surgical glove.

15. Rubber latex article according to claim 13 **characterized in that** the article is a condom.

16. Rubber latex article according to claim 13 **characterized in that** the article is an inflatable balloon.

17. Use of starch for reducing the allergen activity of rubber latex.

18. Use according to claim 17 **characterized in that** the starch is a modified starch.

19. Use according to claim 18 **characterized in that** the modified starch is obtainable by gelatinising the starch in an extruder and subsequently crosslinking the starch with glyoxal.

20. Use according to any of the claims 17, 18 or 19 **characterized in that** the starch is potato starch, Tapioca, waxy corn starch or waxy rice starch.

21. Use of rubber latex according to any of the claims 1-6 for the manufacture of rubber latex articles.
